# EUROPÄISCHE PATENTSCHRIFT

(11) **EP 0 461 559 B1**
(45) Veröffentlichungstag und Bekanntmachung des Hinweises auf die Patenterteilung: **10.09.1997**
(21) Anmeldenummer: 91109368.0
(22) Anmeldetag: 07.06.1991
(51) Int. Cl.: A61K 31/685, A61K 9/127

(54) **Wirkstofffreie Liposomen zur Behandlung von Atherosklerose**
Non active substance containing liposomes for the treatment of atherosclerosis
Liposomes sans agent actif pour le traitement l'athérosclérose

(30) Priorität: 12.06.1990 DE 4018767
(43) Veröffentlichungstag der Anmeldung: 18.12.1991
(73) Patentinhaber: INEX Pharmaceutical Corp., Vancouver B.C. Canada V6P 6G2 (CA)
(72) Erfinder: Schmitt, Jürgen, Dr., W-6313 Homberg/Ohm 3 (DE); Nehne, Jörg, Dr., W-3501 Guxhagen (DE); Feller, Wolfgang, Dr., W-3508 Melsungen (DE)
(74) Vertreter: von Kreisler, Alek, Dipl.-Chem.

(56) Entgegenhaltungen:
- EP-A- 0 234 919
- WO-A-86/01404
- DE-A- 3 815 473
- ARZNEIM-FORSCH, Band 24, Nr. 1, 1974, Seiten 11-16; R.F.A. ALTMAN et al.: "Phospholipids in experimental atherosclerosis"
- J. LIPID. RES., Band 1, Nr. 4, Juli 1960, Seiten 343-348; S.O. BYERS et al.: "Effect of infusions of phosphatides upon the atherosclerotic aorta in situ and as an ocular aortic implant"
- PROCEEDINGS OF THE NATIONAL ACADEMY OF SCIENCES OF USA, Band 85, Januar 1988, Seiten 242-246; K.J. WILLIAMS et al.: "Low density lipoprotein receptor- independent hepatic uptake of a synthetic, cholesterol-scavenging lipoprotein: Implications for the treatment of receptor-deficient atherosclerosis"

## Beschreibung

Bei Dispersionen von Phospholipiden in entsprechenden Konzentrationen in wäßrigen Medien bilden sich Lipiddoppelschichten aus, die sich zu vesikulären Partikeln zusammenschließen. Im einfachsten Fall liegen mehrere Phospholipid-Bilayer konzentrisch umeinander und schließen dabei jeweils einen wäßrigen Zwischenraum ein. Diese Lipidvesikel werden multilamellare Vesikel (MLV) oder allgemein Liposomen genannt. Mit geeigneten Herstellungsmethoden können auch Liposomen hergestellt werden, die aus nur einem Phospholipid-Bilayer bestehen, welches einen wäßrigen Kern einschließt. Vereinbarungsgemäß differenziert man zwischen drei Typen von Liposomen, die sich hinsichtlich ihrer Größe und ihrer Anzahl an Bilayern unterscheiden:
- multilamellar vesicles (MLV):: 0,5 - mehrere µm
- small unilamellar vesicles (SUV):: 0,02 - 0,05 µm
- large unilamellar vesicles (LUV):: 0,06 - 0,5 µm

Man kann die Liposomen als Zweiphasen-Systeme betrachten, wobei eine wäßrige Phase von einer Lipidphase, die aus den Kohlenwasserstoffketten der im Bilayer zusammengelagerten Phospholipide besteht, eingeschlossen wird. Diese Tatsache eröffnete unter anderem die Möglichkeit, sie im therapeutischen Bereich, nämlich als Wirkstoffträger, einzusetzen. Dabei können wasserlösliche Wirkstoffe in die wäßrige Phase und lipidlösliche Wirkstoffe in die Lipidphase der Liposomen eingebaut werden, Med. Phys. 9, 149-175, (1982).

Liposomal verkapselt wurden bisher eine sehr große Anzahl unterschiedlicher Wirkstoffe und Wirkstoffgruppen, wie z.B. Antibiotika (Gentamycin), Steroide (Triamcinolin), Cytostatika (Cisplatin), Fungizide (Amphotericin B), Proteine (Insulin), Anti-Tumormittel (Adriamycin). Die Wirksamkeit dieser wirkstoffhaltigen Liposomen konnte an in vitro-Untersuchungsmodellen und auch in in vivo-Untersuchungen gezeigt werden. So offenbart z. B. die DE-A-3815473 Wirkstoffsysteme, umfassend eine Lipidkomponente mit zumindest einem Transfer-Protein, wobei diese Systeme in Form von Liposomen vorliegen können.

Bei allen aufgeführten Beispielen wird die Fähigkeit der Liposomen ausgenutzt, Wirkstoffe unterschiedlichster Art zu verkapseln und im Blutkreislauf zu transportieren.

Weiterhin is aus EP-A-0 234 919 und WO 86/01404 die Verwendung von wirkstofffreien Phospholipid-Liposomen als Arzneimittel und zur intravenösen Ernährung bekannt.

Seit Anfang der sechziger Jahre wurde in tierexperimentellen Untersuchungen festgestellt, daß es nach Infusionen/Injektionen von in wäßriger Lösung dispergierten Phospholipiden zu einem Anstieg der Konzentrationen an freiem Cholesterin im Serum kommt, Drug Res. 24, 11-16, (1974); J.Lipid Res. 1, 343-348 (1960); J. Path. Bact. 94, 77-87, (1967); J. Lab. & Clin. Med. 56, 30-37, (1960); Artery 1, 106-114, (1975) und FEBS Letters 233, 143-147, (1988). Außerdem wurde gefunden, daß es nach einer Langzeit-Behandlung von atherosklerotischen Kaninchen mit Phospholipid-Dispersionen und mizellaren Phospholipid-Lösungen zur Regression atherosklerotischer Plaques in den Aortenwänden kommt. Weiterhin berichtet Proc. Natl. Acad. Sci., USA, Vol. 85, 242-246, Januar 1988 über Untersuchungen an erblich bedingter Hyperlipidämie bei Kaninchen mit einem ausgeprägten Mangel an LDL-Rezeptoren, denen radioaktiv markierte Phospholipidliposomen injiziert werden. Über die Größe der verwendeten Liposomen wird keine genaue Angabe gemacht, jedoch verweist Seite 243, linke Spalte, 3. Absatz auf einen Sterilisierungsvorgang mittels Filtration durch einen 0,45 µm Filter.

Bei keiner der bekannten Untersuchungen erfolgte eine Analyse der Lipidartikel mit Untersuchungsmethoden wie z.B. Licht- und Elektronenmikroskopie. Aufgrund der in der Literatur beschriebenen Herstellungsmethoden waren die daraus resultierenden Phospholipid-Dispersionen hinsichtlich Aufbau und Größe der Partikel jedoch sehr heterogen.

Ziel der Erfindung ist es, wirksame Mittel zur Behandlung von Atherosklerose zu entwickeln. Die Aufgabe wird durch die Verwendung von unilamellaren Phospholipid-Liposomen zur Herstellung eines Mittels zur Behandlung von Artherosklerose gelöst. Vorzugsweise sind die verwendeten Phospholipid-Liposomen wirkstofffrei und ihre mittlere Teilchengröße beträgt 20 bis 200 nm.

Durch die intravenöse Applikation der Liposomen wird körpereigenes Cholesterin aus den unterschiedlichsten Geweben mobilisiert und aus dem Körper entfernt. Der therapeutische Effekt der wirkstofffreien Phospholipid-Liposomen liegt im Abbau der atherosklerotischen Plaques in den Gefäßwänden.

Bei einem therapeutischen Einsatz der erfindungsgemäßen Phospholipid-Liposomen ist ihre Homogenität in Größe und Aufbau von entscheidender Bedeutung. Bevorzugt wird eine mittlere Teilchengröße der Phospholipid-Liposomen von 50 bis 120 nm, besonders bevorzugt 50 bis 80 nm.

Liposomen vom Typ LUV bzw. SUV sind auch deshalb von Vorteil, weil sie eine wesentlich höhere in vivo und in vitro-Stabilität als Liposomen vom Typ MLV besitzen. Bedingt durch ihre spezielle Herstellungstechnik (z.B. Ultraschall) haben Liposomen vom Typ SUV den Nachteil, daß sie nur mit erheblich größerem Aufwand herzustellen sind und man auch keine so homogene Dispersion wie bei Liposomen des Typs LUV erhält.

Die Phospholipid-Liposomen der vorliegenden Erfindung werden bevorzugt intravenös appliziert.

Erfindungsgemäß werden bevorzugt unilamellare Phospholipid-Liposomen eingesetzt, die aus natürlich vorkommenden Phospholipiden, überwiegend aus Ei- oder Soja-Phospholipiden hergestellt werden. Die eingesetzten Phospholipide enthalten Polyenyl-Fettsäurereste mit 14 - 24 Kohlenstoffatomen.

Die der Erfindung zugrundeliegenden wirkstofffreien Liposomen können aus folgenden Phospholipiden hergestellt werden:
1. Phospholipide und Phospholipidgemische, wie sie aus Soja oder Ei gewonnen werden, z.B. Gemische unterschiedlicher Zusammensetzung aus Phosphatidylcholin und Phosphatidylethanolamin mit einem Anteil geringer Mengen anderer Lipide oder Lipidklassen, wie z.B. neutrale Lipide, Glycolipide und Sphingomyeline.
2. Die unter Pkt. 1 beschriebenen Lipide durch katalytische Hydrierung chemisch modifiziert in unterschiedlichen Hydrierungsgraden.
3. Phospholipide natürlichen Ursprungs, wie sie aus Soja oder Ei und anderen biologischen Materialien gewonnen werden können, so z.B. Phosphatidylcholin (PC), Phosphatidylethanolamin (PE), Phosphatidylglycerol (PG), Phosphatidylinosit (PI), Phosphatidylserin (PS), Phosphatidsäure (PA).
4. Synthetische und halbsynthetische Phospholipide mit definierter Fettsäurezusammensetzung wie z.B. Di-palmitoyl-phosphatidylcholin (DPPC), Di-stearoyl-phosphatidylcholin (DSPC), Stearoyl-palmitoyl-phosphatidylcholin (SPPC), Di-palmitoyl-phosphatidylethanolamin (DPPE), Di-stearoyl-phosphatidylethanolamin (DSPE), Di-myristoyl-phosphatidylserin (DMPS), Di-oleyl-phosphatidylcholin (DOPC) und alle physikalisch möglichen Mischungen der einzelnen Substanzen.

Zusätzlich zu den aufgeführten Phospholipiden können bei der Herstellung wirkstofffreier Liposomen andere Lipide und lipidartige Substanzen als Hilfsstoffe eingesetzt werden. Dazu gehören beispielsweise die als Antioxidantien wirkenden Tocopherole, Tocopherolester und Ascorbylpalmitat und auch Stearylamin und Dicetylphosphat, die zur Erzeugung einer Oberflächenladung auf den Liposomen eingesetzt werden.

Die beschriebenen Liposomen können mit einer ganzen Reihe unterschiedlicher Methoden hergestellt werden.
a) Hochdruckhomogenisierung
b) Extrusion durch Polycarbonatmembranen
c) Mikrofluidisierung
d) Extrusion durch Keramik-Filtermodule
e) Detergens-Dialyse
f) Lösungsmittel-Injektion
g) Reverse-Phase Evaporation-Methode (REV)
h) Calciuminduzierte Fusion
i) Ether-Infusionsmethode
j) Kombination mehrerer Herstellungsmethoden:

1. Hochdruckhomogenisierung mit anschließender Extrusion durch Keramikfilter (d) oder PC-Membranen (b)
2. Mikrofluidisierung mit anschließender Extrusion durch Keramikfilter (d) oder PC-Membranen (b).
   Zur Herstellung von wirkstofffreien Liposomen können als wäßrige Phase bzw. Hydratisierungsmedien neben einfachen Salzlösungen (z.B. physiologische NaCl) alle für einen physiologischen Einsatz geeigneten wäßrigen Lösungen und Puffer benutzt werden (wie z.B. Kohlenhydrat- und Glycerin-haltige Lösungen).

Mit Ausnahme von Herstellungsmethode (h) sind alle aufgeführten Lipide bei den anderen Methoden einsetzbar. Bei den Methoden (a-d) und (j) verläuft die Herstellung der Liposomen generell in zwei Schritten. Im ersten Schritt werden Phospholipide in einem wäßrigen Medium dispergiert. Dazu wird eine entsprechende Menge Phospholipid entweder in einen Rundkolben oder in ein Becherglas eingewogen und mit der wäßrigen Phase (z.B. physiologische Kochsalzlösung, 2,5 %-ige Glycerinlösung, 5 %-ige Sorbitlösung) versetzt. Die Dispergierung des Phospholipids wird so vorgenommen, daß bei Raumtemperatur 2 Stunden geschüttelt wird oder die Dispersion 15 - 30 min am Rotationsverdampfer bei mittlerer Umdrehungszahl bewegt wird. Eine weitere Möglichkeit der Dispergierung besteht darin, Lipid und wäßrige Phase 10 min bei Raumtemperatur mit einem Ultraturrax (bei 5000 Umdrehungen/min) zu behandeln. Bei den so durchgeführten Dispergierungen bilden sich Liposomen vom Typ MLV, die eine sehr große Verteilungsbreite hinsichtlich ihrer Teilchengröße besitzen. Die auf die oben beschriebene Art und Weise hergestellten Phospholipid-Dispersionen werden danach einem der unten aufgeführten Herstellungsprozesse unterworfen.

### a) Hochdruckhomogenisierung

Die Lipid-Dispersion wird in einem handelsüblichen Hochdruckhomogenisator 5 - 50 min bei einem Druck größer als 50 bar umgewälzt.

### b) Extrusion durch Polycarbonat-Membranen

Das dispergierte Phospholipid wird mit Hilfe eines Extruders jeweils mehrmals durch Polycarbonat-Membranen mit unterschiedlichen, kontinuierlich kleiner werdenden Poren bei Drucken zwischen 1 und 40 bar gepreßt.

### c) Mikrofluidisierung

Ein handelsüblicher Mikrofluidizer wird eingesetzt, um aus der Phospholipid-Dispersion die gewünschten Liposomen herzustellen.

Die Dispersion wird dazu in mehreren Zyklen bei Drucken größer als 150 bar homogenisiert.

### d) Extrusion durch Keramikfiltermodule

Nach der Dispergierung des Phospholipids wird die MLV-Dispersion mit Hilfe einer Pumpe so häufig durch einen Keramikfilter mit Porenweiten von 0,2 - 1,0 µm gepumpt, bis die gewünschte Teilchengröße erhalten wird.

### j) Methoden-Kombination

Bei der Kombination von Hochdruckhomogenisierung und Mikrofluidisation mit den beiden Extrusionsverfahren dienen die Extrusionen dazu, die Verteilungsbreiten der Teilchengrößen der Dispersionen zu verbessern.

Bei den folgenden Methoden zur Herstellung von Liposomen des Typs LUV wird nicht über die Vorstufe der MLV-Bildung gegangen:

### e) Detergens-Dialyse

Das Phospholipid und ein dialysierbares Detergens, wie z.B. Na-Cholat oder Octylglucosid werden in einem organischen Lösungsmittel (z. B. CHCl₃) aufgelöst. In einem Rundkolben wird durch Entfernen des Lösungsmittels ein Film gebildet. Der im Vakuum getrocknete Film wird mit dem entsprechenden Hydratisierungsmedium von der Kolbenwand abgelöst. Es bildet sich eine mizellare Lösung, die Mischmizellen aus Phospholipid und Detergens enthält. Das Detergens wird mit Hilfe eines Plattendialysators über eine geeignete Dialysemembran aus der Lösung entfernt und es bilden sich Liposomen vom Typ LUV.

### g) REV-Methode

Die Phospholipide werden in organischen Lösungsmitteln aufgelöst, zu dieser Lösung wird die entsprechende wäßrige Phase gegeben. Durch Behandlung mit Ultraschall wird eine homogene Emulsion hergestellt. Durch Entfernen des organischen Lösungsmittels am Rotationsverdampfer werden die Liposomen gebildet.

### h) Liposomenbildung (LUV) durch Calciuminduzierte Fusion

Diese Methode ist beschränkt auf den Einsatz von sauren Phospholipiden wie z.B. PA, PG und PS. In einem ersten Verfahrensschritt werden zunächst, z.B. durch Ultraschall-Behandlung von Phospholipid-Dispersionen, Liposomen vom Typ SUV gebildet. Durch Zugabe von Ca²⁺ enthaltenden Lösungen fusionieren diese Liposomen zu multilamellaren Vesikeln. Diese Vesikel können durch Zugabe von EDTA in Liposomen vom Typ LUV überführt werden.

### i) Ether-Infusionsmethode

Bei dieser Art der Liposomenherstellung werden die entsprechenden Phospholipide in Diethyl- oder Petrolether aufgelöst. Diese Lösung wird dann in eine wäßrige Phase injiziert, deren Temperatur höher liegt als die Siedepunktstemperatur des benutzten Ethers. Mit dem Verdampfen des Ethers (kann auch im Vakuum bei niedrigerer Temperatur vorgenommen werden) bilden sich Liposomen vom Typ LUV.

Nach der Herstellung der wirkstofffreien Liposomen werden diese routinemäßig hinsichtlich ihrer Größe, Größenverteilung und ihres Aufbaus analysiert.

Zur Bestimmung des mittleren Teilchendurchmessers und der entsprechenden Verteilungsbreite wird die Photonenkorrelationsspektroskopie (PCS) herangezogen. Dazu müssen die Liposomenproben mit dem Hydratisierungsmedium in einen für die Messung günstigen Konzentrationsbereich gebracht werden. In einem handelsüblichen Gerät, z. B. Autosizer II_{c} der Fa. Malvern, wird dann das Laserstreulicht in einem Beobachtungswinkel von 90 ° gemessen. Die gemessenen Streulicht-Intensitäten werden mit Hilfe mathematischer Modelle in mittlere Teilchendurchmesser umgerechnet.

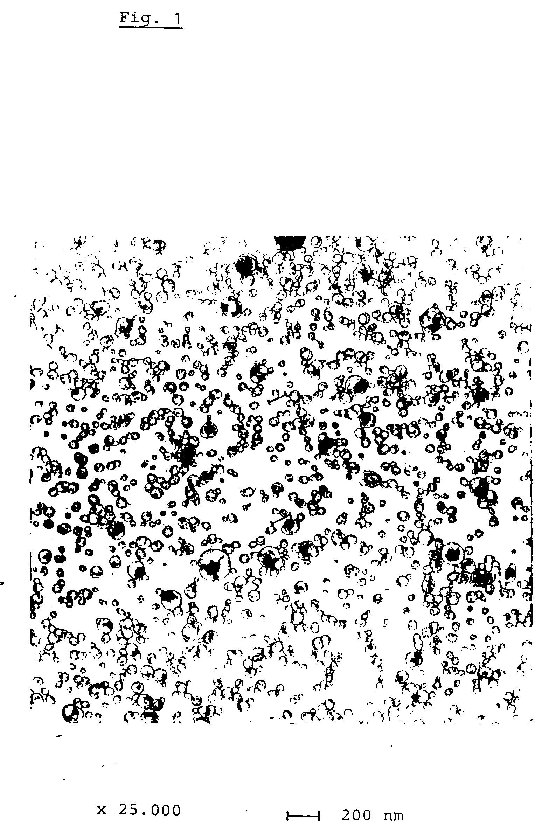

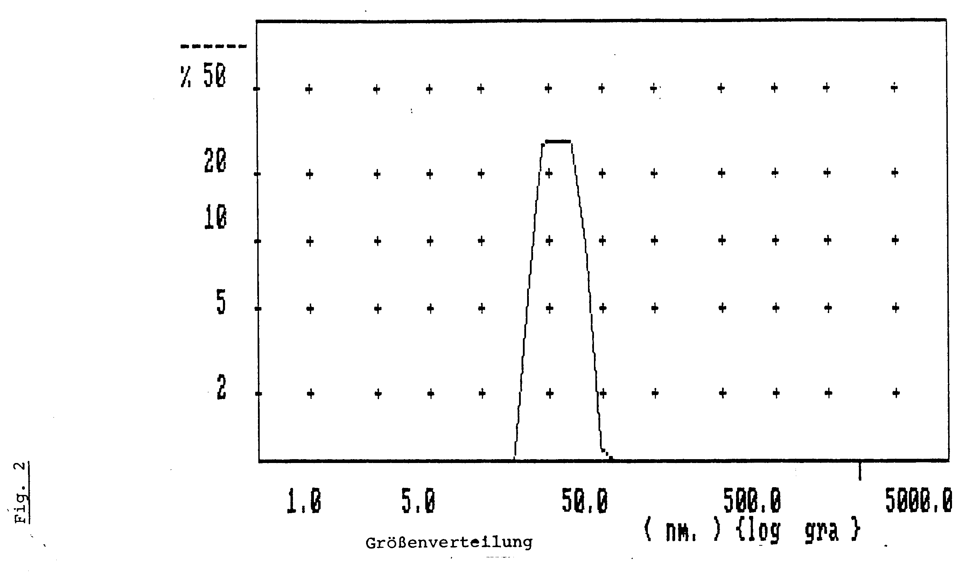

Der Aufbau (Lamellarität) der hergestellten Liposomen wird mittels Transmissionselektronenmikroskopie nach Negativkontrastierung untersucht, Biophys. Acta 557, 9-23 (1979). Die Abbildungen 1 und 2 zeigen eine elektronenmikroskopische Aufnahme sowie die Größenverteilung (PCS-Messung) von Liposomen, die gemäß Beispiel 2 hergestellt wurden.

Das der Erfindung zugrundeliegende Ziel, wirkstofffreie Liposomen zur Behandlung von Atherosklerose und anderer Stoffwechselkrankheiten, wie z. B. Hypercholesterinämie einzusetzen, ist völlig unabhängig von der jeweiligen Herstellungsmethode der verwendeten Liposomen. Mit allen der in den Beispielen aufgeführten Liposomen läßt sich eine Mobilisierung von körpereigenem Cholesterin erzielen.

Sowohl aus den oben geschilderten Gründen der Homogenität und Stabilität der Liposomen-Dispersionen als auch aus Untersuchungen mit gemäß Beispiel 21 hergestellten multilamellaren großen Liposomen (MLV) ergibt sich, daß zur Erreichung des angestrebten therapeutischen Effekts wirkstofffreie Liposomen mit mittleren Teilchengrößen von 50 bis 120 nm am besten geeignet sind.

Wie in Untersuchungen zur cholesterinmobilisierenden und antiatherosklerotischen Wirkung unilamellarer, wirkstofffreier Liposomen gezeigt werden konnte, (Beispiele 21 u. 22) sind Liposomen mit Teilchengrößen unterhalb 200 nm deutlich effektiver als größere. Dabei stellen sich Liposomen als biologisch besonders aktiv heraus, die gemäß Beispiel 13 hergestellt wurden und eine Teilchengröße von nur 60 nm besaßen.

Von Vorteil ist im besonderen auch noch, daß die eingesetzten Phospholipide und die meisten zur Liposomenherstellung benutzten Hilfsstoffe seit vielen Jahren in handelsüblichen Fettemulsionen zur parenteralen Ernährung eingesetzt wurden. Die physiologische Unbedenklichkeit der verwendeten Substanzen ist damit gewährleistet.

Untersuchungen mit i.v.-applizierten, wirkstofffreien Liposomen (hergestellt gemäß den Beispielen 1 - 20) haben deren antiatherosklerotische, cholesterinmobilisierende Wirkung unter Beweis gestellt. Als Untersuchungsmodelle dienten Kaninchen, wobei in einer Versuchsreihe mit einer Standarddiät ernährte Tiere eingesetzt wurden. In der zweiten Versuchsreihe wurden dagegen Tiere eingesetzt, die nach einer 14wöchigen Fütterung mit einer cholesterinreichen Diät (0,3 g Cholesterin/Tag) eine Atherosklerose entwickelt haben. Beiden Untersuchungsgruppen wurden wirkstofffreie Liposomen i.v. appliziert.

Die Ergebnisse der beiden Versuchsreihen zeigen einen deutlichen, therapeutisch nutzbaren Effekt i.v.-applizierter, wirkstofffreier Phospholipid-Liposomen. Durch intravenöse Injektion der erfindungsgemäßen Liposomen wird körpereigenes Cholesterin mobilisiert und im Blutkreislauf in der LDL-Cholesterin-Fraktion transportiert. Neuere Untersuchungen (Perspectives in Biology and Medicine 27, 3 (1984) zeigen, daß das Cholesterin von den wirkstofffreien Liposomen im Austausch gegen Phospholipid unter Vermittlung der Lipoproteine aufgenommen wird. Über den Metabolismus dieser cholesterinreichen liposomen gibt es noch keine genaueren Untersuchungen. In den Tierversuchen wurde aber auch gezeigt, daß eine Behandlung mit wirkstofffreien Liposomen über einen längeren Zeitraum zu einer deutlichen Verminderung atherosklerotischer Ablagerungen in den Gefäßwänden führt. Der Hauptschwerpunkt beim therapeutischen Einsatz der wirkstofffreien Liposomen liegt deshalb in der Regression atherosklerotisch bedingter Gefäßschädigungen. Die Wirksamkeit der Liposomen ist unabhängig von der Methode ihrer Herstellung, so daß alle in den Beispielen aufgeführten Liposomen eingesetzt werden können und zu einem therapeutisch nutzbaren Effekt führen, wobei das Ausmaß der Cholesterinmobilisierung und damit der therapeutische Nutzen bei der Verwendung unilamellarer Liposomen mit einer Partikelgröße unter 120 nm deutlich zunimmt.

### Beschreibung der Figuren

Fig. 1 zeigt eine elektronenmikroskopische Aufnahme der erfindungsgemäßen Liposomen (hergestellt nach Beispiel 2).

Fig. 2 zeigt die Größenverteilung (PCS-Messung) von Liposomen (hergestellt gemäß Beispiel 2).

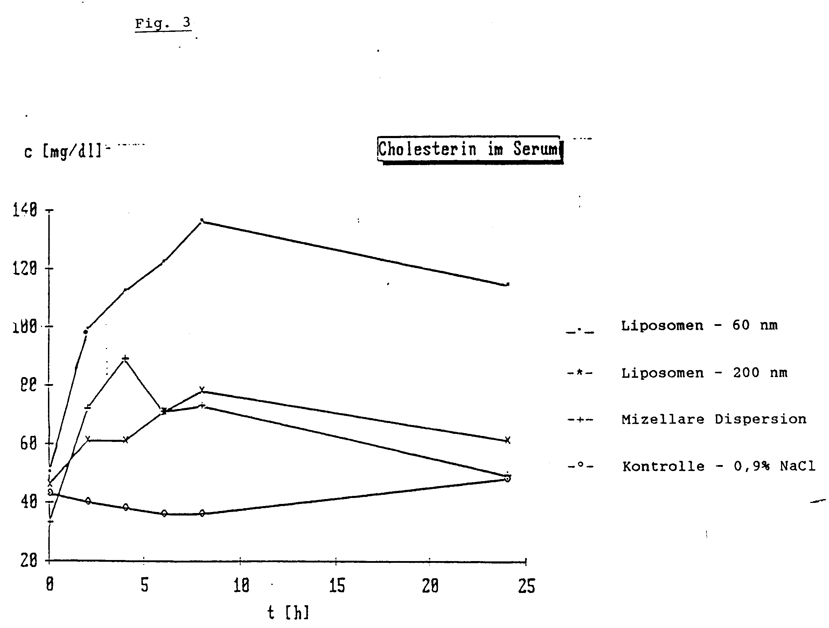

Fig. 3 zeigt den Cholesterin-Gehalt im Serum in Abhängigkeit der Anwesenheit von Liposomen (60 nm), Liposomen (200 nm) und mizellarer Dispersion. Kontrolle ist eine 0,9 %ige NaCl-Lösung.

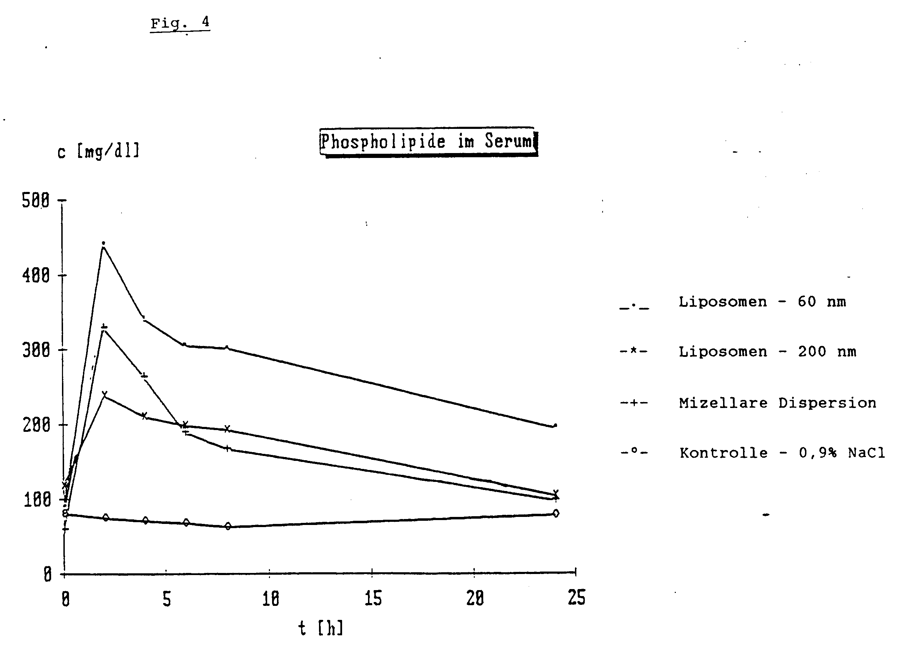

Fig. 4 zeigt den Phospholipid-Gehalt im Serum in Abhängigkeit der Anwesenheit von Liposomen (60 nm), Liposomen (200 nm) und mizellarer Dispersion. Als Kontrolle dient eine 0,9 %ige NaCl-Lösung.

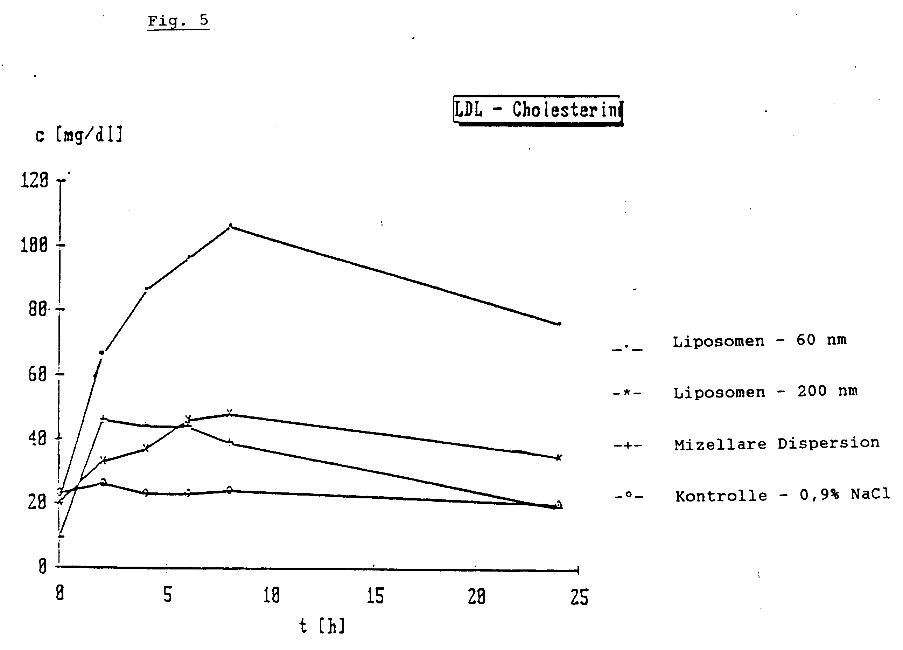

Fig. 5 zeigt den LDL-Cholesterin-Gehalt in Abhängigkeit der Anwesenheit von Liposomen (60 nm), Liposomen (200 nm) und mizellarer Dispersion. Als Kontrolle dient eine 0,9 %ige NaCl-Lösung.

### Beispiele

Die Beispiele 1 bis 20 beschreiben die möglichen Herstellungsverfahren und -bedingungen für die erfindungsgemäßen wirkstofffreien Liposomen.

### Beispiel 1

20 g Soja-Phospholipide werden in einen 2-l-Rundkolben eingewogen, dazu werden 200 ml 0,9 %ige NaCl-Lösung gegeben. An einem Rotationsverdampfer wird das Phospholipid bei 100 Umdrehungen/min 30 min, bei einer Wasserbadtemperatur von 40 °C, vollständig dispergiert.

Danach wird die Dispersion in einen Extruder eingefüllt und bei 20 - 25 °C nacheinander jeweils zehnmal durch Polycarbonatmembranen (Fa. Nuclepore) der folgenden Porenweiten gepreßt: 1,0; 0,8; 0,6; 0,4 und 0,2 µm. Zur Extrusion wird N₂-Gas benutzt, ein maximaler Druck von 10 bar wird nicht überschritten. Im Anschluß an die Extrusion wird durch einen Tiefenfilter (0,2 µm) sterilfiltriert.

### Beispiel 2

100 g Ei-Phospholipide und 269 mg α-Tocopherol werden in einen 2 l-Rundkolben eingewogen und in 500 ml CHCl₃ aufgelöst. Das Lösungsmittel wird im Wasserstrahlpumpenvakuum bei 40 °C abgezogen. Der gebildete Lipidfilm wird zusätzlich 12 Stunden im Ölpumpenvakuum bei 40 °C getrocknet. Der getrocknete Lipidfilm wird mit 200 ml 2,5 %iger Glycerinlösung analog zu Beispiel 1 dispergiert. Die Lipid-Dispersion wird in einem Hochdruckhomogenisator 20 min bei 300 bar homogenisiert. Im Anschluß an die Hochdruckhomogenisierung wird die Dispersion sterilfiltriert.

### Beispiel 3

50 g Ei-Phospholipide werden in ein 200 ml-Becherglas eingewogen. Dazu werden 100 ml 5 %iger Sorbit-Lösung gegeben. Dispergiert wird durch eine 10-minütige Behandlung mit einem Ultraturrax-Gerät bei 5000 Umdrehungen/min. Danach wird mit 5 %iger Sorbitlösung auf ein Gesamtvolumen von 200 ml eingesetllt. Diese Dispersion wird mit einem Mikrofluidizer 10 min bei 600 bar im Umlaufverfahren bearbeitet.

### Beispiel 4

2,0 g Soja-Phospholipide und 5 mg α-Tocopherol werden in einem 500 ml-Rundkolben eingewogen und in CHCl₃ aufgelöst. Nach Abziehen des Lösungsmittels im Wasserstrahlpumpenvakuum wird noch 12 Stunden im Ölpumpenvakuum bei 40 °C getrocknet. Anschließend wird der Lipidfilm mit 200 ml 2,5 %iger Glycerinlösung analog zu Beispiel 1 vollständig dispergiert. Diese Dispersion wird im Hochdruckhomogenisator 5 min bei 600 bar umgewälzt und anschließend sterilfiltriert.

### Beispiel 5

40 g Ei-Phospholipide und 107 mg α-Tocopherol werden in ein Becherglas eingewogen und 100 ml 2,5 %ige Glycerinlösung dazugegeben. Die Dispergierung wird mit einem Ultraturrax vorgenommen (10 min, 5000 U/min), danach wird mit Glycerinlösung auf 200 ml aufgefüllt. Die Dispersion wird in einen Extruder umgefüllt und genau wie in Beispiel 1 behandelt.

### Beispiel 6

250 mg DPPC werden zusammen mit 319 mg Oktylglucosid in einen 250 ml-Rundkolben eingewogen (Lipid/Detergens-Verhältnis = 0,3) und in 50 ml CHCl₃ aufgelöst. Das Lösungsmittel wird im Ölpumpenvakuum getrocknet. Nach Zugabe von 10 ml physiologischer Kochsalzlösung entsteht eine klare Lösung. Diese Lösung wird 4 h lang in einem Plattendialysator gegen 0,9 %ige Kochsalzlösung dialysiert.

### Beispiel 7

250 mg Soja-Phospholipide werden zusammen mit 430 mg Na-Cholat (Lipid-Detergens-Verhältnis = 1,0) in CHCl₃ aufgelöst. Der getrocknete Lipidfilm (Beispiel 6) wird in 2,5 % Glycerinlösung aufgenommen und 4 h gegen die gleiche Lösung dialysiert.

### Beispiel 8

1,0 g Ei-Phospholipide und 2,5 mg α-Tocopherol werden zusammen mit 1,2 g Na-Cholat in CHCl₃ aufgelöst (Lipid-/Detergens-Verhältnis = 1,0). Nach Abziehen des Lösungsmittels und Trocknung des Lipidfilms wird diese in 10 ml 5 %iger Sorbitlösung aufgenommen, diese Lösung wird 4 h dialysiert.

### Beispiel 9

100 g Ei-Phospholipide, 5 g Stearylamin und 100 mg α-Tocopherol werden in einem 2 l-Rundkolben in CHCl₃ aufgelöst. Nach Abdampfen des Lösungsmittels wird der Lipidfilm im Vakuum getrocknet und in 1 l 0,9 %iger NaCl-Lösung am Rotationsverdampfer bei 40 °C 2 h dispergiert. Diese Dispersion wird mit Hilfe einer Pumpe so oft durch ein Keramikfiltermodul (Ceraflo™) mit einer Porenweite von 0,2 µm gefördert, bis sich die gewünschte Teilchengröße eingestellt hat.

### Beispiel 10

4,0 g Phosphatidylcholin (PC) und 0,2 g Dicetylphosphat werden in 100 ml Ethanol aufgelöst. Diese Lösung wird mittels einer Spritze in 1,5 l 0,9 %ige NaCl-Lösung gespritzt. Die entstehenden Liposomen werden durch Ultrafiltration bis auf eine Phospholipid-Konzentration von 50 mg/ml ankonzentriert.

### Beispiel 11

2,5 g Ei-Phospholipide und 2,5 g hydrierte Ei-Phospholipide werden in einem 2 l-Rundkolben in 600 ml eines Gemisches aus Isopropylether/Chloroform (1 : 1) aufgelöst. Dazu werden 100 ml einer wäßrigen 5 %igen Sorbitlösung gegeben. Diese Präparation wird 1 h in einem Ultraschallbad behandelt, bis eine homogene Emulsion entstanden ist. Aus dieser Emulsion wird das Lösungsmittel am Rotationsverdampfer abgezogen, worauf sich die Liposomen bilden.

### Beispiel 12

100 mg Soja-Phospholipide werden in 30 ml Diethylether gelöst. Diese Lösung wird mit Hilfe eines Infusomaten in eine 60 °C warme 0,9 %ige NaCl-Lösung mit einer Geschwindigkeit von 0,2 ml/min injiziert. Gleichzeitig mit dem Verdampfen des Lösungsmittels bilden sich unilamellare Phospholipid-Liposomen.

### Beispiel 13

30 g Ei-Phospholipide werden in ein Becherglas eingewogen, dazu werden 200 ml 0,9 %ige NaCl-Lösung gegeben. Das Phospholipid wird mit einem Ultraturrax 10 min bei 5000 U/min dispergiert. Die Dispersion wird mit 0,9 %iger NaCl-Lösung auf 300 ml aufgefüllt. Danach wird in einem Hochdruckhomogenisator bei 45 °C und 560 bar 30 min homogenisiert. Anschließend wird die Dispersion mit Hilfe eines Extruders zehnmal durch eine Polycarbonatmembran der Porenweite 0,050 µm gepreßt und sterilfiltriert.

### Beispiel 14

45 g Ei-Phospholipide werden in ein Becherglas eingewogen. Nach Zugabe von 350 ml 0,9 %iger NaCl-Lösung wird 10 min mit einem Ultraturrax bei 5000 U/min dispergiert. Die Dispersion wird anschließend in einem Hochdruckhomogenisator 5 min bei Raumtemperatur und 500 bar behandelt und danach sterilfiltriert.

### Beispiel 15

45 g Ei-Phospholipide werden unter den gleichen Bedingungen wie in Beispiel 14 bearbeitet. Nach der Hochdruckhomogenisierung wird die Dispersion mehrmals (mindestens 10 Umläufe) durch einen Keramikfilter (Ceraflo™) mit der Porenweite 0,2 µm gepumpt und anschließend sterilfiltriert.

### Beispiel 16

35 g Ei-Phospholipide werden mittels eines Ultraturrax bei 5000 U/min 5 min in 2,5 %iger Glycerin-Lösung in 10 m M Tris x HCl, pH 7,5, dispergiert. Die Dispersion wird mindestens 10mal durch einen Keramikfilter der Porenweite 0,2 µm gepumpt und anschließend sterilfiltriert.

### Beispiel 17

100 g Ei-Phospholipide und 269 mg α-Tocopherol werden gemäß Beispiel 2 behandelt. Nach der Hochdruckhomogenisierung wird die Dispersion entweder mit Hilfe eines Extruders zehnmal durch eine Polycarbonatmembran der Porenweite 0,1 µm oder mit einer Pumpe durch einen Keramikfilter der Porenweite 0,2 µm gepumpt.

### Beispiel 18

2,0 g Ei-Phospholipide werden in einem 500 ml-Rundkolben mit CHCl₃ aufgelöst. Das Lösungsmittel wird im Wasserstrahlpumpenvakuum entfernt und der entstandene Lipidfilm 12 h im Ölpumpenvakuum bei maximal 40 °C getrocknet. Der Lipidfilm wird in 150 ml 0,9 %iger NaCl-Lösung gemäß Beispiel 1 dispergiert. Diese Dispersion wird in einem Mikrofluidizer 5 min bei Raumtemperatur und 850 bar behandelt und anschließend sterilfiltriert.

### Beispiel 19

2,0 g Ei-Phospholipide werden gemäß Beispiel 18 bearbeitet. Nach der Mikrofluidisierung wird die Dispersion entweder mittels eines Extruders zehnmal durch eine Polycarbonatmembran der Porenweite 0,1 µm gepreßt oder zehnmal durch einen Keramikfilter mit der Porenweite 0,2 µm gepumpt. Das Endprodukt wird dann sterilfiltriert.

### Beispiel 20

50 g Ei-Phospholipide werden gemäß Beispiel 3 bearbeitet, nach der Mikrofluidisierung wird mit der Dispersion analog zu Beispiel 19 verfahren.
In den Beispielen 21 und 22 wird die Anwendung der wirkstofffreien Liposomen als Mittel zur Mobilisierung von Cholesterin und zur Regression atherosklerotischer Plaques beschrieben.

### Beispiel 21

In einem Tierexperiment wird die Wirkung von Liposomen, hergestellt gemäß den Beispielen 13 und 15, mit einer handelsüblichen mizellaren Phospholipid-Dispersion und einer MLV-Präparation verglichen. Die vier Dispersionen werden je 6 mit standarddiäternährten Kaninchen in gleichen Dosierungen intravenös appliziert, eine Kontrollgruppe (n = 6) erhält physiologische Kochsalzlösung. Vor der Injektion und zu 5 Zeitpunkten nach der Injektion werden Blutentnahmen vorgenommen. Folgende Serumparameter werden bestimmt: Gesamtcholesterin-Konzentration, LDL-Cholesterin-Konzentration, Phospholipid-Konzentration. Die untersuchten Proben hatten folgende Partikelgrößen: Liposomen, Beispiel 13 = 60 nm; Liposomen, Beispiel 15 = 200 nm; mizellare Dispersion = < 10 nm; MLV-Präparation = > 500 nm. In den Skizzen 1 - 3 ist der zeitliche Verlauf der Konzentrationen der untersuchten Serumparameter dargestellt. Daraus geht eindeutig hervor, daß die Liposomen mit einer Partikelgröße von 60 nm hinsichtlich der cholesterinmmobilisierenden Wirkung am effektivsten sind. Nicht nur die Konzentration für das Gesamtcholesterin im Serum, sondern auch die Konzentration der LDL-Cholesterin-Fraktion steigen gegenüber den anderen getesteten Dispersionen bei den 60 nm-Liposomen überdurchschnittlich stark an. Die Höhe und der zeitliche Verlauf der Serum-Phospholipid-Konzentration zeigt, daß die 60 nm-Liposomen deutlich länger im Blutkreislauf zirkulieren und damit auch länger therapeutisch nutzbar sind.

### Beispiel 22

Zu diesen Untersuchungen wurden atherosklerotische Kaninchen eingesetzt (n = 60). Die Atherosklerose wird in den Kaninchen durch eine 14wöchige Ernährung mit einer cholesterinreichen Diät (0,3 g Cholesterin/Tag) erzeugt. Nach Abschluß dieser Fütterungsbzw. Atherosklerosephase haben die Tiere einen Plasmacholesterin-Spiegel von ca. 1000 mg/dl. Danach wird die cholesterinreiche Diät abgesetzt, innerhalb von 6 Wochen sinken die Cholesterin-Plasmaspiegel auf 200 - 300 mg/dl (Randomisierungsphase). Die Tiere werden in eine Kontrollgruppe und 2 Testgruppen zu je 20 Tieren eingeteilt. Die Tiere der Testgruppen erhalten intravenöse Applikationen von Liposomen, die gemäß Beispiel 13 hergestellt wurden (Regressionsphase).

Folgende Dosierungen wurden ausgewählt:
Gruppe 1: 50 mg Ei-Phospholipid/kg Körpergewicht = ca. 1,0 g/Woche
Gruppe 2: 200 mg Ei-Phospholipid/kg Körpergewicht = ca. 2,0 g/Woche
Gruppe 3: Kontrollgruppe, physiologische NaCl

Die entsprechenden Volumina an Liposomen und physiologischer NaCl wurden 2x wöchentlich in die Ohrrandvene injiziert. Der gesamte Behandlungszeitraum betrug 16 Wochen. Danach wurden die Tiere aller drei Gruppen getötet, die Aorten entnommen, planimetrisch und densitometrisch analysiert. Als Vergleich wurden zusätzlich noch die Aorten von 20 normal ernährten Kaninchen untersucht. Mit Hilfe der rechnergesteueurten Analysenmethode wurde das Ausmaß der atherosklerotischen Gefäßschädigungen gemäß der Einteilung von Friedman et al (Lit. 14) vorgenommen.

Die Ergebnisse (Tabelle 1) zeigen einen deutlichen, dosisabhängigen antiatherosklerotischen Effekt der wirkstofffreien Liposomen. Es kommt zu einer signifikanten Verringerung der atherosklerotischen Plaques, besonders ausgeprägt bei der hohen Dosierung.

**Tabelle 1**

| | Anzahl der Tiere | Grad der Atherosklerose |
|---|---|---|
| normal ernährte Tiere | 20 | 0 |
| Gruppe 1 | 18 | 1,8 (0,6 - 2,9) |
| Gruppe 2 | 17 | 1,1 (0,0 - 3,3) |
| Gruppe 3 | 19 | 4,6 (4,2 - 5) |

## Patentansprüche

1. Verwendung von unilamellaren Phospholipid-Liposomen zur Herstellung eines Mittels zur Behandlung von Atherosklerose.

2. Verwendung nach Anspruch 1, dadurch gekennzeichnet, daß die Phospholipid-Liposomen wirkstofffrei sind und ihre mittlere Teilchengröße 20 bis 200 nm beträgt.

3. Verwendung nach Anspruch 2, dadurch gekennzeichnet, daß die mittlere Teilchengröße der Phospholipid-Liposomen 50 bis 120 nm, besonders bevorzugt 50 bis 80 nm beträgt.

4. Verwendung nach einem der Ansprüche 1 bis 3, dadurch gekennzeichnet, daß die unilamellaren Phospholipid-Liposomen aus natürlich vorkommenden Phospholipiden, bevorzugt aus Ei- oder Sojaphospholipiden hergestellt werden.

5. Verwendung nach Anspruch 4, dadurch gekennzeichnet, daß die Phospholipide Polyenyl-Fettsäurereste mit 14 bis 24 C-Atomen enthalten.

6. Verwendung nach Anspruch 5, dadurch gekennzeichnet, daß die Phospholipide aus einem Phospholipidgemisch aus Phosphatidylcholin und Phosphatidylethanolamin mit einem Anteil geringerer Mengen anderer Lipide oder Lipidklassen bestehen.

7. Verwendung nach einem der Ansprüche 1 bis 3, dadurch gekennzeichnet, daß die Phospholipid-Liposomen besonders aus synthetischen und halbsynthetischen Phospholipiden mit definierter Fettsäurezusammensetzung hergestellt sind, ausgewählt aus der Gruppe Di-palmitoyl-phosphatidylcholin, Di-stearoyl-phosphatidylcholin, Stearoyl-palmitoyl-phosphatidylcholin, Di-palmitoyl-phosphatidylethanolamin, Di-stearoyl-phosphatidylethanolamin, Di-myristoyl-phosphatidylserin, Di-oleyl-phosphatidylcholin sowie alle möglichen physikalischen Mischungen der einzelnen Substanzen.

8. Verwendung nach einem der Ansprüche 1 bis 7, dadurch gekennzeichnet, daß die Phospholipid-Liposomen aus Phospholipiden bestehen, die durch katalytische Hydrierung aus den Phospholipiden der Ansprüche 4 bis 7 entstanden sind.

9. Verwendung nach einem der Ansprüche 1 bis 8, dadurch gekennzeichnet, daß die Phospholipid-Liposomen weitere Lipide oder lipidartige Substanzen als Hilfsstoffe enthalten.

10. Verwendung nach Anspruch 9, dadurch gekennzeichnet, daß die Hilfsstoffe Antioxidanzien sind, die aus der Gruppe Tocopherole, Tocopherolester und Ascorbylpalmitat ausgewählt sind.

11. Verwendung nach Anspruch 9, dadurch gekennzeichnet, daß die Hilfsstoffe ausgewählt sind aus der Gruppe Stearylamin und Dicetylphosphat.

## Claims

1. Use of unilamellar phospholipid liposomes for the preparation of an agent for the treatment of atherosclerosis.

2. The use according to claim 1, characterized in that said phospholipid liposomes are free of active ingredients and their average particle size is from 20 to 200 nm.

3. The use according to claim 2, characterized in that the average particle size of said phospholipid liposomes is from 50 to 120 nm, particularly preferred from 50 to 80 nm.

4. The use according to any of claims 1 to 3, characterized in that said unilamellar phospholipid liposomes have been prepared from naturally occurring phospholipids, preferably from egg or soybean phospholipids.

5. The use according to claim 4, characterized in that said phospholipids contain polyenyl fatty acid residues having from 14 to 24 carbon atoms.

6. The use according to claim 5, characterized in that said phospholipids consist of a phospholipid mixture of phosphatidylcholine and phosphatidylethanolamine with a minor fraction of other lipids or lipid classes.

7. The use according to any of claims 1 to 3, characterized in that said phospholipid liposomes have been prepared, in particular, from synthetic and semisynthetic phospholipids having a defined fatty acid composition and being selected from the group consisting of dipalmitoylphosphatidylcholine, distearoylphosphatidylcholine, stearoylpalmitoylphosphatidylcholine, dipalmitoylphosphatidylethanolamine, distearoylphosphatidylethanolamine, dimyristoylphosphatidylserine, dioleylphosphatidylcholine, and all possible physical mixtures of these individual substances.

8. The use according to any of claims 1 to 7, characterized in that said phospholipid liposomes consist of phospholipids derived from the phospholipids as defined in claims 4 to 7 through catalytic hydrogenation.

9. The use according to any of claims 1 to 8, characterized in that said phospholipid liposomes contain further lipids or lipoids as adjuvants.

10. The use according to claim 9, characterized in that said adjuvants are antioxidants selected from the group consisting of tocopherols, tocopherol esters and ascorbylpalmitate.

11. The use according to claim 9, characterized in that said adjuvants are selected from the group consisting of stearylamine and dicetyl phosphate.

## Revendications

1. Utilisation de liposomes unilamellaires de phospholipides pour la préparation d'une substance destinée au traitement de l'athérosclérose.

2. Utilisation selon la revendication 1, caractérisée en ce que les liposomes de phospholipides sont sans agent actif et que la taille moyenne de leurs particules est de 20 à 200 nm.

3. Utilisation selon la revendication 2, caractérisée en ce que la taille moyenne des particules des liposomes de phospholipides est de 50 à 120 nm, de façon particulièrement préférée de 50 à 80 nm.

4. Utilisation selon l'une des revendications 1 à 3, caractérisée en ce que les liposomes unilamellaires de phospholipides sont préparés à partir de phospholipides d'origine naturelle, de préférence à partir de phospholipides de l'oeuf ou du soja.

5. Utilisation selon la revendication 4, caractérisée en ce que les phospholipides contiennent des restes de polyène-acide gras ayant de 14 à 24 atomes de carbone.

6. Utilisation selon la revendication 5, caractérisée en ce que les phospholipides sont formés par un mélange de phospholipides à base de phosphatidylcholine et de phosphatidyléthanolamine avec une proportion de quantités moins importantes d'autres lipides ou classes de lipides.

7. Utilisation selon l'une des revendications 1 à 3, caractérisée en ce que les liposomes de phospholipides sont préparés en particulier à partir de phospholipides synthétiques et semi-synthétiques avec une composition d'acide gras définie, choisis dans le groupe di-palmitoyl-phosphatidylcholine, di-stéaroyl-phosphatidylcholine, stéaroyl-palmitoyl-phosphatidylcholine, di-palmitoyl-phosphatidyléthanolarnine, di-stéaroyl-phosphatidyléthanolamine, di-myristoyl-phosphatidylsérine, di-oléyl-phosphatidylcholine ainsi que tous les mélanges physiques possibles des substances prises individuellement.

8. Utilisation selon l'une des revendications 1 à 7, caractérisée en ce que les liposomes de phospholipides sont formés à partir de phospholipides qui ont été créés par hydrogénation catalytique à partir des phospholipides des revendications 4 à 7.

9. Utilisation selon l'une des revendications 1 à 8, caractérisée en ce que les liposomes de phospholipides contiennent d'autres lipides ou substances de type lipidique en tant qu'agents de soutien.

10. Utilisation selon la revendication 9, caractérisée en ce que les agents de soutien sont des antioxydants, qui sont choisis dans le groupe des tocophérols, tocophérolester et ascorbylpalmitate.

11. Utilisation selon la revendication 9, caractérisée en ce que les agents de soutien sont choisis dans le groupe stéarylamine et dicétylphosphate.
